# FASCICULE DE BREVET EUROPEEN

(11) **EP 0 559 508 B1**
(45) Date de publication et mention de la délivrance du brevet: **08.01.1997**
(21) Numéro de dépôt: 93400322.9
(22) Date de dépôt: 09.02.1993
(51) Int. Cl.: A61L 27/00

(54) **Nouveau revêtement pour système prothétique**
Neue Beschichtung für Prothesen
Novel coating for prosthesis

(30) Priorité: 06.03.1992 FR 9202702
(43) Date de publication de la demande: 08.09.1993
(73) Titulaire: ZIMMER S.A., F-94401 Vitry-sur-Seine (FR)
(72) Inventeur: Duquet, Bruno, Appartement 325, F-59000 Lille (FR); Daculsi, Guy, F-44360 Vigneux de Bretagne (FR); Delecrin, Joel, F-44000 Nantes (FR)
(74) Mandataire: Hirsch, Marc-Roger

(56) Documents cités:
- EP-A- 0 371 491
- WO-A-86/06617
- DE-A- 4 020 598
- US-A- 4 103 002
- US-A- 4 542 539
- DATABASE WPIL Week 3288, Derwent Publications Ltd., London, GB; AN 88-224917; & JP-A-63 160 666
- DATABASE WPIL Week 3288, Derwent Publications Ltd., London, GB; AN 88-224916; & JP-A-63 160 665

## Description

La présente invention a pour objet un nouveau revêtement pour système prothétique ainsi que les systèmes prothétiques recouverts avec le présent revêtement.

Les prothèses ou systèmes prothétiques sont de plus en plus utilisés dans le domaine de la chirurgie. Des exemples de telles applications concernent l'utilisation en orthopédie de prothèses du fémur et de ma hanche en cas de fracture du col du fémur, d'arthrose de la hanche, les prothèses pour la chirurgie réparatrice maxillo-faciale, l'orthopédie et autres.

Comme tout corps étranger introduit dans l'organisme humain ou animal, le système prothétique doit être intégré par l'organisme, mais doit aussi remplir les fonctions de l'organe déficient ou remplacé, et ceci de façon rapide, sûre et durable.

Les matériaux dits biocompatibles couramment utilisés pour la fabrication de ces systèmes prothétiques destinés à être implantés sont les céramiques, les alliages Co-Cr-Mo, et surtout actuellement le titane et ses alliages. Grâce à de tels matériaux biocompatibles, le système prothétique osseux n'est que très peu sujet au phénomène de rejet. Néanmoins, des problèmes spécifiques se sont posés. En effet, dans la mesure où ces systèmes prothétiques osseux sont implantés dans un os, par exemple une prothèse de fémur dans le canal intramédullaire, il est nécessaire afin que le système prothétique remplissent ses fonctions de façon efficace, rapide, sûre et durable qu'il y ait croissance rapide, sûre et durable de l'os à l'interface autour de ce système prothétique et subséquemment ancrage ("Bone Bonding") à celui-ci. Ainsi, il a été proposé des revêtements pour systèmes prothétiques dont la fonction est de promouvoir ladite croissance ou réponse osseuse à l'interface os/prothèse pour conduire à une apposition osseuse et une ostéo-intégration, et ceci de façon rapide, sûre et durable.

De nombreux revêtements ont été proposés parmi lesquels on trouve les microbilles, les céramiques alumineuses, les bio-verres et autre.

PCT/EP86/00268 décrit un revêtement poreux biocompatible, caractérisé par une couche de base en titane, elle-même formée d'une couche dense et d'une couche rugueuse, puis une couche intermédiaire, puis une couche de surface extrêmement dense.

DE 4 020 598 décrit un système prothétique à base de céramique, comprenant une première couche de verre, puis une couche d'un matériau à base de phosphate de calcium.

EP-A-0 371 491 décrit un système prothétique à base de métal, comprenant une couche externe imperméable aux ions métalliques et aux fluides corporels, à base de céramique et phosphate de calcium.

Les revêtements qui ont jusqu'à présent été retenus sont à base de phosphate de calcium. En effet, l'os contenant une proportion majoritaire de phosphate de calcium, il a naturellement été employé des revêtements dont la nature et la structure chimique se rapprochent de celles de l'os. Ces différents phosphates de calcium sont l'hydroxyapatite (HA), le phosphate tricalcique (TCP) sous forme α et β (respectivement α-TCP et β-TCP) et quelques autres phosphates de calcium. Ces phosphates de Ca se différencient par leur stoechiométrie et leurs propriétés cristallographiques. Actuellement, le phosphate de calcium se rapprochant le plus du type osseux est l'hydroxyapatite. Ce dernier composé est maintenant largement utilisé en tant que revêtement de système prothétique, avec des épaisseurs variant de quelques dizaines à plusieurs centaines de µm. Les composés tels que le TCP et les mélanges de celui-ci avec l'HA sont aussi utilisés, ainsi que d'autres phosphates de calcium.

Malheureusement, la croissance osseuse étant un phénomène extrêmement complexe, les revêtements prothétiques phosphocalciques actuels ne donne pas actuellement entière satisfaction. En effet, la réponse osseuse doit se faire au dépend du matériau; le matériau doit, par conséquent, avoir des propriétés originales de résorption, substitution, échange d'ions. On décrit ces propriétés sous le terme de bio-activité. Seuls certains matériaux comme les bio-verres et les phosphates de calcium présentent ces propriétés de bio-activité qui permettent l'ostéogénèse par résorption/substitution du revêtement. La croissance de l'os par résorption/substitution peut être décomposée en deux grandes réactions: une réaction chimique et une réaction histologique.

La réaction histologique comprend tout d'abord une étape de "nettoyage" effectuée à l'aide de macrophages qui débarrassent la surface de l'implant par phagocytose.

Ensuite, la réaction histologique se poursuit par une colonisation du revêtement par des cellules, telles que des ostéoblastes, fibroblastes, les macrophages précédemment cités, etc., et par du liquide extra-cellulaire. Il y a colonisation des surfaces du revêtement. Les composants extra-cellulaires contiennent en outre des fibres protéiques, telles que des fibres collagéniques. Lorsque la matrice s'organise et est principalement constituée des fibres collagènes, elle est alors hautement différenciée et constitue un ostéoïde.

La réaction chimique comprend tout d'abord une dissolution extra-cellulaire. La solution comprise dans le revêtement s'enrichit en ions calcium et phosphore, libérés par la dissolution de certains cristaux. Ensuite, les ions libérés et dissous précipitent. Cette précipitation a lieu dans le liquide extra-cellulaire, un milieu riche en protéines sur lesquelles se fixent les ions. Les cristaux ainsi formés sont des cristaux aciculaires d'apatites biologiques identiques à ceux de l'os. Lorsque la matrice protéique est un ostéide, la précipitation des ions conduit à une ostéocoalescence entre le tissu hôte et la surface du matériau prothétique. L'ostéogénèse à la surface du revêtement ainsi obtenue se caractérise par un os vrai, formé à la surface et au dépend du matériau bioactif constitutif de la prothèse.

Divers revêtements ont été proposés et le plus couramment employé est l'HA. Ce revêtement présente cependant l'inconvénient ne n'être que très lentement résorbable et peu bioactif. Certains auteurs ont décrit l'HA comme un matériau non résorbable, c'est-à-dire que la dégradation selon le processus de dissolution/précipitation est très lente et, de plus, l'HA ne favorise pas la minéralisation sur la matrice collagénique, but ultime recherché. La formation et l'apposition d'un os vrai pourraient être retardées. On a donc proposé des revêtements plus bioréactifs que l'HA, c'est-à-dire semi-résorbable. Parmi ceux-ci, le TCP est le plus bio-réactif. Malheureusement, la biodégradation du TCP est parfois trop rapide, la résorption étant trop importante. L'ostéoïde n'est pas encore formé lors de la dissolution/précipitation et donc il ne peut se former d'os. Il apparaît nécessaire d'avoir un produit mixte associant la stabilité du HA et la forte bioréactivité du TCP, comme dans les applications d'un matériau biphasé macroporeux en comblement osseux.

Ainsi, il a été proposé des systèmes biphasés HA/TCP ou BCP. Les BCP de proportion 60/40 répondent aux objectifs d'équilibre entre les phénomènes histologiques et chimiques. Néanmoins, le BCP présente toujours deux inconvénients. Comme la technique de dépôt du revêtement est la torche à plasma ou l'électrophorèse associé à un frittage, la forme cristalline du TCP peut varier entre la forme β et α, l'α-TCP est plus soluble que le β, et les propriétés du BCP varient en conséquence. Le BCP obtenu par plasma est donc très bioactif. En cas de complications secondaires, telles que la mobilisation de l'implant dans l'os receveur ou des complications septiques, ces complications pourraient entraîner la mise à nu rapide de la surface du matériau biocompatible constitutif de la prothèse. Il se produit donc une encapsulation fibreuse à court terme et il ne pourrait y avoir ostéo-intégration.

Ainsi, aucun revêtement actuellement utilisé ne permet de remplir efficacement les objectifs visés que sont une apposition osseuse et un ancrage de façon rapide, sûre et durable.

La présente invention a donc pour objet un nouveau revêtement bioactif pour système prothétique avec une cinétique de dégradation/substitution et d'apposition osseuse améliorée.

La présente invention fournit donc un revêtement pour système prothétique caractérisé en ce qu'il présente un gradient de résorption/substitution selon le sens perpendiculaire à la surface dudit système prothétique, la couche interne étant la moins résorbable.
Le terme "résorption/substitution" signifie dans la présente description la capacité d'un matériau à se résorber -ou se dégrader- par le phénomène de précipitation/dissolution et le processus concomitant de remplacement par du tissu osseux. Une résorption/substitution élevée correspond à une résorption rapide, et inversement.
Le terme "gradient de résorption/substitution" désigne dans la présente description et dans les revendications auxquelles elle donne lieu, une différence, aussi bien discrète que continue, de la résorption/substitution entre les couches externe et interne, la couche interne étant la moins résorbable. La couche interne est la couche au contact du matériau biocompatible constitutif de la prothèse, la couche externe étant au contact du tissu hôte, c'est-à-dire l'os receveur. Cette différence est d'un facteur d'au moins 3, et cette différence peut être monotone ou non.

Le revêtement est constitué de matériau phosphocalcique, c'est-à-dire de phosphate de calcium.

Selon un mode de réalisation, le revêtement est constitué de 2 ou plusieurs couches successives de résorption/substitution différentes présentant ledit gradient de résorption/substitution. La différence est dans ce cas discrète.

Avantageusement, le revêtement est constitué d'une couche à résorption/substitution élevée et d'une couche à résorption/substitution faible.

Ladite couche de résorption/substitution faible est de préférence en un matériau non-résorbable, c'est-à-dire très peu résorbable et peu bioactif. Avantageusement, ledit matériau non-résorbable est l'hydroxyapatite (HA).
Ladite couche à résorption/substitution élevée est de préférence en un matériau semi-résorbable. Avantageusement, ledit matériau semi-résorbable est un biphasé HA/TCP. Ledit biphasé HA/TCP est un biphasé de composition massique 30/70 à 80/20.

Selon le mode de réalisation dans lequel le présent revêtement est constitué de deux couches, l'épaisseur de ladite couche à résorption/substitution élevée est de 10 à 100 µm, de préférence 20 à 50 µm.

Selon ce même mode de réalisation, l'épaisseur de ladite couche à résorption/substitution faible est de 1 à 40 µm, de préférence 1 à 10 µm.

La présente invention a aussi pour objet tout système prothétique recouvert du présent revêtement. Ce système prothétique peut être une prothèse de fémur, maxillo-faciale, etc. L'application du revêtement selon la présente invention se fait par tout procédé classique, tel que électrophorèse avec frittage, ou à l'aide d'une torche à plasma.

Les exemples suivants sont donnés à titre illustratif de la présente invention, susceptible de variantes aisément accessibles à l'homme de l'art.

### EXEMPLE

L'évaluation des propriétés est obtenue grâce à une étude statistique sur 40 lapins. Cette évaluation est mise en oeuvre sur des éprouvettes simples de type bâtonnet cylindrique soumises à des tests d'extraction axiale et à des analyses de coupes histologiques.

### Revêtements et surfaces testés

- Tr:: témoin dont le revêtement est résorbé (Ra: 2,2)
- Ts:: témoin dont l'état de surface est sablé à la bille de verre (Ra: 0,7)
- HA:: hydroxyapatite à 100% ± 3% (impuretés) (50 µm)
- BCP:: biphasé 60% HA/40% TCP (50 µm)
- RS:: revêtement selon la présente invention:
- HA 100% ± 3%, 30 µm
- BCP 60/40, 30 µm
Les revêtements sont appliqués à l'aide d'une torche à plasma. Les conditions opératoires sont les conditions classiquement utilisées pour une torche atmosphérique.

### Eprouvettes

La forme de l'éprouvette est étudiée pour faciliter son extraction et son implantation. L'éprouvette est cylindrique, de longueur 9 mm, de diamètre extérieur 3,5 mm, et percée d'un évidement intérieur circulaire de diamètre 1 mm. L'éprouvette est en titane TA 6V (selon la norme ASTM F 620.79).

### Implantation des éprouvettes

Chaque animal, de sexe masculin, a le même poids (2,7 ± 0,2 kg) lors de la première intervention.

L'implantation a lieu à deux endroits. Le premier est situé dans la zone épiphysaire supérieure du tibia (au-dessous du "genou" et dans le plan frontal) et le second est situé dans la zone épiphysaire inférieure du fémur (au-dessus du "genou" et dans le plan sagittal). L'implantation chirurgicale se fait par voie interne, en prenant soin de déplacer les masses musculaires pour permettre un recouvrement ultérieur. Le forage s'effectue à vitesse constante, limité par un foret (⌀ 3,5 mm) à longueur prédéterminée et sous irrigation constante. L'éprouvette est alors introduite dans le trou de forage ainsi réalisé. Le contrôle de la pression de forage est manuel et l'appréciation de l'incidence est visuelle.

### Mode d'analyse statistique

40 lapins sont utilisés aux fins d'analyse. La première intervention consiste à implanter 2 échantillons sur une patte postérieure. Trois semaines plus tard, la deuxième patte reçoit également 2 implants. Le sacrifice a lieu au bout de 6 semaines permettant ainsi de récupérer sur un même lapin des implants de 3 et 6 semaines qui subiront des tests d'extraction et des analyses de coupes histologiques. Chaque type d'éprouvette est représenté 6 fois dans chaque site d'implantation et pour chaque délai d'implantation. Cette répartition est résumée dans le tableau I.

**TABLEAU I**

| EPROUVETTES | 3 SEMAINES | | | | 6 SEMAINES | | | | TOTAUX | |
|---|---|---|---|---|---|---|---|---|---|---|
| | FEMUR | | TIBIA | | FEMUR | | TIBIA | | HISTO | Extraction |
| | HISTO* | Extraction | HISTO | Extraction | HISTO | Extraction | HISTO | Extraction | | |
| HA | 1 | 5 | 1 | 5 | 1 | 5 | 1 | 5 | 4 | 20 |
| BCP | 1 | 5 | 1 | 5 | 1 | 5 | 1 | 5 | 4 | 20 |
| RS | 1 | 5 | 1 | 5 | 1 | 5 | 1 | 5 | 4 | 20 |
| Ts | 1 | 5 | 0 | 12 | 0 | 6 | 1 | 11 | 2 | 34 |
| Tr | 0 | 12 | 1 | 5 | 1 | 11 | 0 | 6 | 2 | 34 |
| | | | | | | | | | 16 | 128 |

| | | | | | | | | | | |
|---|---|---|---|---|---|---|---|---|---|---|
| * : coupe histologique | | | | | | | | | | |

### Extraction des éprouvettes

Les implants qui subissent des tests d'extraction suivent le mode opératoire suivant:
- dissection des os,
- dégagement des extrémités des éprouvettes avec un scalpel,
- congélation immédiatement après, dans l'azote liquide,
- mise en place des os dans un gabarit,
- extraction des éprouvettes par repoussage: l'extrémité d'une pièce de guidage cylindrique de 3,5 mm de diamètre est mise en contact avec une extrémité de l'éprouvette,
- relevé des forces nécessaires à l'extraction de chaque éprouvette,
- calcul des contraintes après définition de la surface active d'après radiographie, la radiographie étant mise en oeuvre sur des clichés gradués.
Les résultats sont donnés en MPa dans le tableau II ci-après.

**TABLEAU II**

| | 3 SEMAINES | | 6 SEMAINES | |
|---|---|---|---|---|
| | FEMUR | TIBIA | FEMUR | TIBIA |
| HA | 2,760 | 3,053 | 3,025 | 2,439 |
| BCP | 3,214 | 3,251 | 3,199 | 2,582 |
| RS | 4,090 | 3,846 | 3,574 | 5,262 |
| Tr | 2,144 | 3,238 | 2,438 | 2,165 |
| Ts | 0,520 | 0,641 | 0,840 | 1,382 |

Ces résultats montrent clairement que la force d'extraction pour le revêtement selon la présente invention est nettement supérieure aux revêtements classiques.

### Coupes histologiques

Les implants qui subissent les études histologiques suivent le mode opératoire suivant:
- euthanasie des lapins et dissection immédiate,
- observation clinique au cours de la dissection des masses musculaires,
- prélèvement du segment fémoral contenant les implants par section au disque diamanté ou à la scie à os,
- fixation au mélange gluta-paraformaldéhyde,
- radiographie X des pièces sur films standards dentaires,
- déshydratation des pièces par alcool de degrés croissants,
- inclusion des pièces méthylméthacrylate selon le procédé des tissus calcifiés,
- polymérisation à l'étuve,
- coupes à la tronçonneuse à disque diamanté,
- amincissement par disque abrasif,
- réalisation de microradiographies de contact sur film HR,
- montage entre lames et lamelles,
- observations en microscopie photonique en lumière normale, polarisée et en lumière bleue pour l'étude fluorescence,
- étude d'une coupe par implant en MEB et en micro-analyse en dispersion d'énergie de l'interface os/prothèse.

### Résultats histologiques

### - Témoin sablé: Ts

Une repousse osseuse est observée, venant très souvent jusqu'au contact osseux. Un fin liseré fibreux ou ostéoïde sépare l'os de l'implant. Un remaniement osseux est observé dès la 6ème semaine.

### - Revêtement résorbé: Tr

L'état de surface de ces échantillons est très rugueux (grenaillé). La repousse osseuse semble meilleure que pour le titane sablé, l'interface fibreux ostéoïde est rarement visible. Des processus de remaniement haversiens sont visibles au contact direct de l'implant.

### - Revêtement HA

La réponse osseuse est importante et "court" le long de l'implant depuis la corticale jusque sur quelques dixièmes de mm en zone médullaire. Le remaniement osseux apparaît dès la 3ème semaine et bien que le contact osseux soit parfait, il persiste des zones médullaires au contact du revêtement HA. Dans ces zones, semblent apparaître des processus de résorption, mais très limités.

### - Revêtement BCP

La repousse osseuse est très importante dès la 3ème semaine, l'ensemble de l'implant est recouvert d'os. En zone médullaire, on observe un front continu d'os de 50 à 100 µm d'épaisseur, lamellaire, bien minéralisé, riche en ostéocytes.

Des zones de résorptions apparaissent, et la "texture du revêtement" a changé. En lumière polarisée, à fort grandissement, il semble y avoir une infiltration par des composants de matrice organique et une transformation du minéral, des pores sont visibles (quelques microns de diamètre).

A 6 semaines, la densité osseuse est accrue, et de nombreux remaniements osseux sont observés jusqu'au contact du BCP. Le dépôt semble être cependant très fragile, friable, car de nombreux artéfacts de préparation apparaissent.

### - Revêtement sandwich: RS

Les deux couches ne sont pas identifiables. Le résultat histologique est identique à celui des éprouvettes ayant eu un revêtement BCP; cependant, les artéfacts de préparation et de coupes sont moins apparents.

La résistance du revêtement sandwich au contraire semble donc meilleure que pour le BCP seul, et similaire à celui du revêtement HA, mais par contre la qualité de la repousse et sa quantité (en particulier en zone médullaire) est similaire au revêtement BCP.

Le revêtement sandwich permet ainsi de:
- développer une forte bioactivité (résorption/substitution osseuse) et une ostéoconduction meilleure que HA,
- conserver une tenue mécanique aux contraintes équivalentes aux dépôts HA classiques.

## Revendications

1. Revêtement phosphocalcique pour système prothétique présentant un gradient de résorption/substitution selon le sens perpendiculaire à la surface dudit système prothétique d'au moins 3, la couche interne étant la moins résorbable, et comprenant:
(i) une couche interne d'hydroxyapatite; et
(ii) une couche externe d'un biphasé hydroxyapatite/phosphate tricalcique.

2. Revêtement selon la revendication 1, dans lequel ledit biphasé hydroxyapatite/phosphate tricalcique est un biphasé de composition massique 30/70 à 80/20.

3. Revêtement selon la revendication 1 ou 2, qui comprend plusieurs couches successives possédant une résorption/substitution différente présentant ledit gradient de résorption/substitution.

4. Revêtement selon l'une quelconque des revendications 1 à 3, dans lequel l'épaisseur de ladite couche (ii) de biphasé hydroxyapatite/phosphate tricalcique est de 10 à 100 µm.

5. Revêtement selon l'une quelconque des revendications 1 à 4, dans lequel l'épaisseur de ladite couche (ii) de biphasé hydroxyapatite/phosphate tricalcique est de 20 à 50 µm.

6. Revêtement selon l'une quelconque des revendications 1 à 5, dans lequel l'épaisseur de ladite couche (i) d'hydroxyapatite est de 1 à 40 µm.

7. Revêtement selon l'une quelconque des revendications 1 à 6, dans lequel l'épaisseur de ladite couche (i) d'hydroxyapatite est de 1 à 10 µm.

8. Système prothétique recouvert du revêtement selon l'une quelconque des revendications 1 à 7.

## Claims

1. A phospho-calcium coating for a prothesis system having a resorption/substitution gradient in the direction perpendicular to the surface of said prophesis system of at least 3, the inner layer being the least resorbable, and comprising:
(i) an inner hydroxyapatite layer; and
(ii) an outer two-phase hydroxyapatite/tricalcium phosphate layer

2. Coating according to claim 1, wherein said two-phase hydroxyapatite/tricalcium phosphate has a two-phase composition by mass of 30/70 to 80/20.

3. Coating according to claim 1 or 2 comprising several successive layers exhibiting different resorption/substitution having said resorbtion/substitution gradient.

4. Coating according to any one of claims 1 to 3, wherein the thickness of said two-phase hydroxyapatite/tricalcium phosphate layer (ii) is from 10 to 100 µm.

5. Coating according to any one of claims 1 to 4, wherein the thickness of said two-phase hydroxyapatite/tricalcium phosphate layer (ii) is from 20 to 50 µm.

6. Coating according to any one of claims 1 to 5, wherein the thickness of said hydroxyapatite layer (i) is from 1 to 40 µm.

7. Coating according to any one of claims 1 to 6, wherein the thickness of said hydroxyapatite layer (i) is from 1 to 10 µm.

8. A prothesis system covered with the coating according to any one of claims 1 to 7.

## Patentansprüche

1. Phosphorkalziumhaltige Beschichtungn für ein Prothesensystem, das einen Resorptions-/Substitutionsgradienten senkrecht zur Oberfläche des genannten Prothesensystems mit einem Wert von mindestens 3 aufweist, wobei die innere Schicht diejenige mit dem geringsten Resorptionsvermögen ist, bestehend aus
(i) einer Hydoxyapatit-Innenschicht sowie
(ii) einer zweiphasigen Außenschicht aus Trikalzium-Hydroxyapatit/-Phosphat.

2. Beschichtung gemäß Anspruch 1, in welcher das genannte zweiphasige Trikalzium-Hydroxyapatit/-Phosphat eine Zweiphasen-Zusammensetzung mit einem Volumenverhältnis von 30/70 bis 80/20 ist.

3. Beschichtung gemäß Anspruch 1 oder 2, die aus mehreren aufeinanderfolgenden Schichten besteht, welche ein unterschiedliches Resorptions-/Substitutionsvermögen mit dem genannten Resorptions-/Substitutionsgradienten aufweisen.

4. Beschichtung gemäß einem der Ansprüche 1 bis 3, in der der Dicke der genannten Schicht (ii) aus zweiphasigem Trikalzium-Hydroxyapatit/-Phosphat 10 bis 100 µm beträgt.

5. Beschichtung gemäß einem der Ansprüche 1 bis 4, in der die Dicke der genannten Schicht (ii) aus zweiphasigem Trikalzium-Hydroxyapatit/-Phosphat 20 bis 50 µm beträgt.

6. Beschichtung gemäß einem der Ansprüche 1 bis 4, in der die Dicke der genannten Schicht (i) aus Hydroxyapatit 1 bis 40 µm beträgt.

7. Beschichtung gemäß einem der Ansprüche 1 bis 6, in der die Dicke der genannten Schicht (i) aus Hydroxyapatit 1 bis 10 µm beträgt.

8. Prothesensystem, das mit einer Beschichtung gemäß einem der Ansprüche 1 bis 7 versehen ist.
